# EUROPEAN PATENT APPLICATION

(11) **EP 2 738 173 A1**
(43) Date of publication of application: **04.06.2014**
(21) Application number: 12306474.3
(22) Date of filing: 28.11.2012
(51) Int. Cl.: C07D 491/04, A61K 31/407, A61K 31/4355, C07D 495/04, A61P 35/00, A61P 5/00, A61P 37/00

(54) **Heterocyclic compounds as inhibitors of the sodium iodide symporter**

(71) Applicant: COMMISSARIAT A L'ENERGIE ATOMIQUE ET AUX ENERGIES ALTERNATIVES, 75015 Paris (FR)
(72) Inventor: Ambroise, Yves, 91940 Les Ulis (FR); Lacotte, Pierre, 34500 Beziers (FR)
(74) Representative: Mena, Sandra

(57) **Abstract**

The invention relates to new compounds of formula (I): 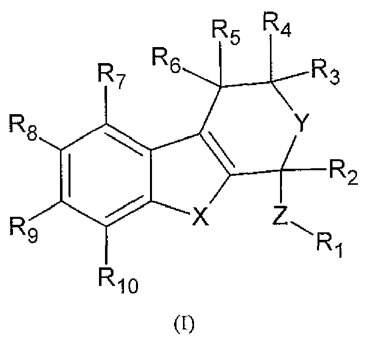 for their use as medicaments, and in particular as inhibitors of sodium iodide symporter (NIS) and reducers of iodine transport and/or accumulation into NIS-expressing cells. The invention also relates to a pharmaceutical composition comprising at least one compound of formula (I) as active principle. Finally, the present invention relates to specific compounds of formula (I) as such.

## Description

The invention relates to new compounds of formula (I) for their use as medicaments, and in particular as inhibitors of sodium iodide symporter (NIS) and reducers of iodine transport and/or accumulation into NIS-expressing cells. The invention also relates to a pharmaceutical composition comprising at least one compound of formula (I) as active principle. Finally, the present invention relates to specific compounds of formula (I) as such.

The transport of iodide from blood into the thyroid gland is essential for the biosynthesis of thyroid hormones T3 and T4. These iodinated hormones are responsible of many vital mechanisms in vertebrates such as metabolism regulation and central nervous system development (S. P. Porterfield et al., Endocr. Rev., 1993, 14, 94-106). Iodide capture by the thyroid gland is mediated by the sodium iodide symporter (NIS), an integral membrane glycoprotein located at the basolateral side of thyrocytes. The molecular characterization of NIS was carried out after cloning the rat and human forms in 1996 (G. Dai, Nature, 1996, 379, 458-460; P. A. Smanik, Biochem. Biophys. Res. Commun., 1996, 226, 339-345). NIS is essentially expressed in thyroid follicular cells and also in several other tissues including the salivary glands, gastric mucosa, and the lactating mammary glands.

Other monovalent anions such as ClO₄⁻, SCN⁻, BF₄⁻, PF₆⁻, NO₃⁻ can also be transported by NIS (J. Wolff, Physiol. Rev., 1964, 44, 45-90; P. A. Jones, Toxicology in vitro, 1996, 10, 149-160). They competitively inhibit iodide transport in rat thyroid-derived cells (FRTL5) with IC₅₀ values of 0.14, 14, 0.75, 0.009, and 250 µM, respectively (F. Waltz et al., Anal. Biochem., 2010, 396, 91-95). Thorough biochemical analysis has clarified the mechanism of iodide uptake and revealed the key role of NIS in many thyroid as well as extra-thyroid diseases such as cancer (thyroid, breast...) (T. Kogai et al., Endocr. Relat. Cancer, 2006, 13, 797-826), autoimmune diseases (Hashimoto and Basedow-Graves' diseases), toxic nodules, thyroiditis, multinodular goiter, etc. (O. Dohan et al., Endocr. Rev., 2003, 24, 48-77). The prevalence rate of these thyroid-related disorders is close to 7% in Western countries. In addition, in case of nuclear accident, the entrapment of radioactive isotopes of iodide by the thyroid gland is a major source of concern since this accumulation is directly responsible for an increase of cancer incidence. Dramatic examples of this are the Chernobyl (1986) and Fukushima (2011) accidents (G. Brumfiel et al., Nature, 2011, 471, 273-275). The consequences of a nuclear reactor breakdown are tragic and it is urgent to find solutions to prevent and treat radioactive contamination. One solution is to develop radioprotective small molecules capable of blocking radioiodide uptake and/or, even better, enabling chemoremediation after the contamination has occurred. On the other hand, the ability of the thyroid gland to accumulate radioiodine has long provided the basis for the diagnosis and treatment of thyroid disorders (E. L. Mazzaferri, The thyroid: a fundamental and clinical text 7th ed.; Braverman, L. E.; Utiger R. D. Eds; Lippincott-Raven: Philadelphia, 1996; pp. 922-945). It is today proposed to extend this strategy to extra-thyroid tissue for the diagnosis and destruction of cancer cells by ¹³¹I after targeted NIS gene transfer (D. P. Carvalho et al., Arq. Bras. Endocrinol. Metabol., 2007, 51, 672-682; C. Spitzweg et al., Clin. Endocrinol., 2002, 57, 559-574). In this case, compounds increasing radioiodide retention in NIS-expressing cell would be very useful to ensure strong and specific toxic effect (N. Lecat-Guillet et al., ChemMedChem, 2008, 3, 1211-1216; T. Kogai et al., Endocr. Relat., Cancer, 2006, 13, 797-826). Small molecules affecting NIS function are unique tools for the study and treatment of many thyroid as well as non-thyroid dysfunctions.

Recently, a high throughput screening led to the discovery of new potent iodide uptake blockers (**ITB-1** to **ITB-10,** Scheme 1) (N. Lecat-Guillet et al., ChemBioChem, 2008, 9, 889-895). These compounds showed rapid and total inhibition of iodide transport using isotopic flux measurement in human embryonic kidney cells stably expressing the human NIS (hNIS-HEK293) as well as in rat thyroid-derived cell lines (FRTL5) with inhibitory concentration values (IC₅₀) in the nano- and micromolar ranges. This inhibition was further confirmed by measurement of iodide-induced current in hNIS-expressing oocytes from *Xenopus laevis.* The IC₅₀ value of Compounds **1** and **2** were reported to be 0.4 µM and 0.3 µM in FRTL5 cells. Further analysis showed that Compounds **1** and **2** are not cytotoxic at concentration up to 200 µM. The discovery of Compounds **1** and **2** as a powerful iodide uptake inhibitor is particularly attractive because tetrahydro-β-carbolines are small versatile structures which can be easily synthesized at low cost and on a large scale. This compound template is found in many natural products, drugs and drugs candidates. A second family of NIS inhibitors is derived from **ITB-9.** Chemical optimization and structure-activity investigation on ITB9 core has led to the recent discovery of nano- to subnanomolar NIS inhibitors (P. Lacotte et al., ChemMedChem, doi: 10.1002/cmdc.201200417). Besides of these two classes of inhibitors discovered by HTS, another small molecule, called **ITB-11** was identified as an iodide uptake inhibitor in FRTL5 cells with an IC₅₀ value of 0.4 µM (N. Lecat-Guillet et al., ChemMedChem, 2008, 3, 1207-1209). **ITB-11** was discovered from rational design, because it shares structural similarities with the NIS substrate BF₄⁻.

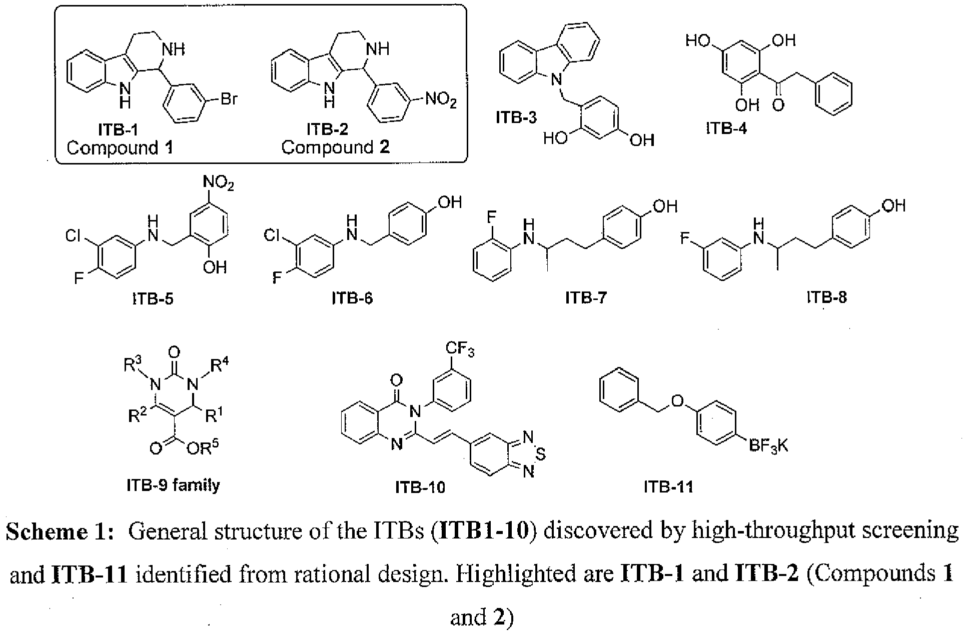

The inventors have now surprisingly discovered a new class of compounds with improved effects on iodide uptake in FRTL5 cells by measuring their IC₅₀ values. This new class of compounds presents a strong enhancement of bioactivity for the inhibition of NIS function compared to Compounds **1** and **2.** The compounds of the invention are thus more suitable for *in vivo* application.

Besides, no chemical compounds are currently available to combat contamination with radioisotopes of iodine, against the adverse effects of an over-accumulation of cold iodine (in some cases of hyperthyroidism and thyrotoxicosis), and for use in functional imaging of NIS.

Therefore, a first subject of the invention is a compound of formula (I) below: 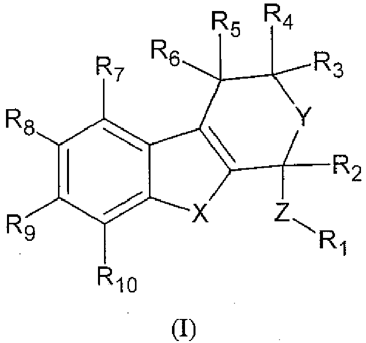 or a pharmaceutically acceptable salt thereof, wherein:
● X = O, S or NR, wherein R is a hydrogen atom or an optionally substituted C₁-C₂₀ linear or branched alkyl chain or an optionally substituted C₃-C₇ cycloalkyl chain, said alkyl or cycloalkyl chain possibly including one or more heteroatoms or carbonyl functions,
● Y = O, S or NH,
● Z is a simple bond or an optionally substituted C₂-C₆ linear or branched alkyl or alkenyl chain or an optionally substituted C₃-C₆ cycloalkyl chain, preferably Z is a simple bond or a C₂ double bond, and still more preferably Z is a simple bond,
● R₁ is an optionally substituted aromatic ring,
● R₂, R₃, R₄, R₅, R₆, R₇, R₈, R₉ and R₁₀, identical or different, are selected from hydrogen atom, optionally substituted C₁-C₂₀ linear or branched alkyl, alkoxy, alkenyl, alkynyl, cycloalkyl, aryl, arylalkyl, heteroalkyl, heteroaryl and heteroarylalkyl groups, and wherein at least one of X or Y is O or S,
for its use as a medicament.

In the sense of the present invention:
- Alkyl groups are chosen among (C₁-C₂₀)alkyl groups, and preferably (C₁-C₆)alkyl groups such as methyl, ethyl, n-propyl, isopropyl, n-butyl, sec-butyl, *tert*-butyl, isobutyl, pentyl and hexyl radicals;
- Cycloalkyl groups refer to a monovalent cyclic hydrocarbon radical preferably of 3 to 7 ring carbons. The cycloalkyl group can have one or more double bonds and can optionally substituted. The term "cycloalkyl" includes, for examples, cyclopropyl, cyclohexyl, cyclohexenyl and the like;
- Heteroalkyl groups mean alkyl groups as defined above in which one or more hydrogen atoms to any carbon of the alkyl, or one carbon atom of the alkyl chain, is replaced by a heteroatom selected from the group consisting of N, O, P, B, S, Si, Sb, Al, Sn, As, Se and Ge. The bond between the carbon atom and the heteroatom may be saturated or unsaturated. Suitable heteroalkyl groups include cyano, benzoyl, methoxy, acetamide, borates, sulfones, sulfates, thianes, phosphates, phosphonates, silanes and the like;
- Alkoxy groups are chosen among (C₁-C₂₀)alkoxy groups, and preferably (C₁-C₄)alkoxy groups such as methyloxy, ethyloxy, n-propyloxy, iso-propyloxy, n-butyloxy, sec-butyloxy, *tert*-butyloxy and isobutyloxy radicals;
- Aryl groups means any functional group or substituent derived from at least one simple aromatic ring; an aromatic ring corresponding to any planar cyclic compound having a delocalized π system in which each atom of the ring comprises a p-orbital, said p-orbitals overlapping themselves. More specifically, the term aryl includes, but is not limited to, phenyl, biphenyl, 1-naphthyl, 2-naphthyl, anthracyl, pyrenyl, and the substituted forms thereof;
- Heteroaryl groups means any functional group or substituent derived from at least one aromatic ring as defined above and containing at least one heteroatom selected from P, S, O and N. The term heteroaryl includes, but is not limited to, furan, pyridine, pyrrole, thiophene, imidazole, pyrazole, oxazole, isoxazole, thiazole, isothiazole, tetrazole, pyridazole, pyridine, pyrazine, pyrimidine, pyridazine, benzofurane, isobenzofurane, indole, isoindole, benzothiophene, benzo[c]thiophene, benzimidazole, indazole, benzoxazole, benzisoxazole, benzothiazole, quinoline, isoquinoline, quinoxaline, quinazoline, cinnoline, purine and acridine. The aryl and heteroaryl groups of the invention comprise preferably 1 to 12 carbon atoms, and more preferably 5 or 6 carbon atoms;
- Arylalkyl means any group derived from an alkyl group as defined above wherein a hydrogen atom is replaced by an aryl or a heteroaryl group.

When the chains or groups of the invention are optionally substituted, the substituants may be selected for example from halogen, hydroxyl, cyano, nitro, carboxylate, carboxyester, amino, ketone, C₁-C₁₂ alkyl, heteroalkyl or alkoxy groups, C₃-C₇ cycloalkyl group, C₁-C₁₂ aryl or heteroalkyl groups.

According to the invention, halogen atoms are chosen among bromine, chlorine, fluorine and iodine, and preferably bromine, chlorine and fluorine.

According to the invention, heteroatoms are chosen among N, O, P, B, S, Si, Sb, Al, Sn, As, Se and Ge, and preferably N, O and S.

According to a preferred embodiment, X = NH.

According to another preferred embodiment, Y = O.

More preferably, R₁ is an optionally substituted aryl or heteroaryl ring, and when the aryl or heteroaryl ring is substituted it is advantageously substituted in position 3. Said aryl ring may be a phenyl ring, for example substituted with at least one halogen atom, nitro (NO₂), amino (NH₂) or C₁-C₆ alkoxy group.

According to a preferred embodiment, R₁ is a 2,3-(methylenedioxy)-phenyl group or a phenyl ring substituted with at least one halogen atom, nitro (NO₂), amino (NH₂) or C₁-C₆ alkoxy group, and still more preferably R₁ is a 2,3-(methylenedioxy)-phenyl group or a phenyl substituted with at least one amino (NH₂) group.

According to a preferred embodiment, R₂, R₃, R₄, R₅, R₆, R₇, R₈, R₉ and R₁₀ are hydrogen atoms.

The present invention also concerns the compound of formula (I) for use as a medicament:
- for the inhibition of sodium iodide symporter (NIS), and the reduction of iodine transport and/or accumulation into NIS-expressing cells,
- for the *in vivo* diagnosis of NIS pathologies by functional imaging,
- for radioiodide decontamination of humans or animals after exposure to radioactive iodine species,
- for the prevention and/or the treatment of thyroid disorders, and more particularly of hyperthyroidism triggered by iodine overload, thyrotoxicosis, thyroiditis and toxic nodular goiter,
- for the prevention and/or the treatment of cancers, and more particularly of thyroid and breast cancers,
- for the prevention and/or the treatment of autoimmune diseases, and more particularly of Hashimoto and Basedow-Graves' diseases.

Functional imaging of NIS is a method of detecting or measuring changes in the spatial distribution of NIS within the body. To achieve this, the compound of formula (I) of the invention needs to be derivatized into a probe with similar chemical and biological characteristics, plus a chemical tag for detection. For the chemical tag, it is generally used radioisotopes such as carbon-11, nitrogen-13, oxygen-15 and fluorine-18, for use in Positron Emission Tomography (PET); technetium-99m for use in Single-Photon Emission Computed Tomography (SPECT).

Another subject matter of the invention is a pharmaceutical composition comprising at least one compound of formula (I) of the invention as an active principle, and at least one pharmaceutically acceptable excipient.

The expression "pharmaceutically acceptable excipient" refers to any diluents, adjuvants or vehicles, such as preserving agents, fillers, disintegrating agents, wetting agents, emulsifying agents, suspending agents, solvents, dispersion media, coatings, antibacterial and antifungal agents, isotonic and absorption delaying agents and the like.

The pharmaceutical composition of the present invention may be administered by any suitable route, for example, by oral, buccal, inhalation, sublingual, nasal, percutaneous, *i.e.* transdermal or parenteral (including intravenous, intramuscular, subcutaneous and intracoronary) administration. Therefore, the pharmaceutical composition of the invention can be provided in various forms, such as in the form of hard gelatin capsules, of capsules, of compressed tablets, of suspensions to be taken orally, of lozenges or of injectable solutions or in any other form appropriate to the method of administration.

The pharmaceutical composition according to the invention includes those wherein a compound of formula (I) is administered in an effective amount to achieve its intended purpose. Determination of the effective amounts is well within the capability of those skilled in the art.

A "therapeutically effective dose" refers to that amount of compound of formula (I) which results in achieving the desired effect. Toxicity and therapeutic efficacy of compound of formula (I) can be easily determined by standard pharmaceutical procedures in cell cultures or experimental animals, *i.e*. for determining the LD₅₀ (the dose lethal to 50% of the population) and the ED₅₀ (the dose therapeutically effective in 50% of the population). The dose ratio between toxic and therapeutic effects is the therapeutic index, which is expressed as the ratio between LD₅₀ and ED₅₀. The data obtained from such data can be used in formulating range of dosage for use in humans. The dosage of compound of formula (I) preferably lies within a range of circulating concentrations that include the ED₅₀ with little or no toxicity. The dosage can vary within this range depending upon the dosage form employed, and the route of administration.

The exact formulation, route of administration, and dosage can be chosen by the individual physician in view of the patient's conditions. Dosage amount and interval of administration can be adjusted individually to provide plasma levels of compound of formula (I) which are sufficient to maintain the preventive or therapeutic effects.

The amount of pharmaceutical composition administered will therefore depend on the subject being treated, on the subject's weight, the severity of the affliction and the manner of administration.

For human and other mammal use, the compounds of formula (I) can be administered alone, but they are preferably administered in admixture with at least one pharmaceutically acceptable carrier, the nature of which will depend on the intended route of administration and the presentation form. Pharmaceutical composition for use according to the present invention thus can be formulated in a conventional manner using one or more physiologically acceptable carriers comprising one or more excipient(s) and/or auxiliary(ies) that facilitate processing of the compounds of formula (I) into preparations which can be used pharmaceutically. Amongst the excipients and auxiliaries which can be used in the pharmaceutical composition according to the invention, one can mention anti-agglomerating agents, preservatives agents, dyes, vitamins, inorganic salts, taste-modifying agents, smoothing agents, coating agents, isolating agents, stabilizing agents, wetting agents, anti-caking agents, dispersing agents, emulsifying agents, aromas, penetrating agents, solubilizing agents, etc., mixtures thereof and generally any excipient conventionally used in the pharmaceutical industry.

By way of example, when the pharmaceutical composition is administered orally, the carrier may comprise one or several excipients such as talc, lactose, starch or modified starches, cellulose or cellulose derivatives, polyethylene glycols, acrylic acid polymers, gelatin, magnesium stearate, animal or vegetal fats of natural or synthetic origin, paraffin derivatives, glycols, etc.

For general information about the formulation and administration of pharmaceutical compositions, one can obviously refer to the book "Remington's Pharmaceutical Sciences", last edition. Of course, a person skilled in the art will take care on this occasion that the excipient(s) and/or auxiliary(ies) optionally used are compatible with the intrinsic properties attached to the pharmaceutical composition in accordance with the invention.

These pharmaceutical compositions can be manufactured in a conventional manner, *i.e.* by conventional mixing, dissolving, granulating, dragee-making, emulsifying, encapsulating, entrapping or lyophilizing processes. Proper formulation is dependent upon the route of administration chosen.

Finally, the invention also concerns compounds of formula (I) as such, responding to the following formulae:

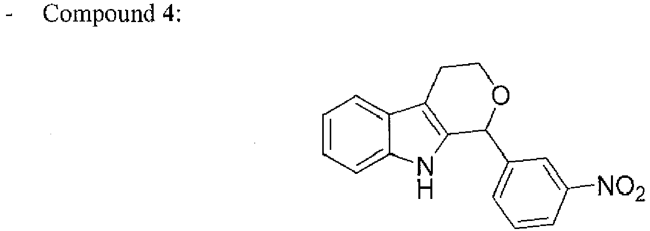

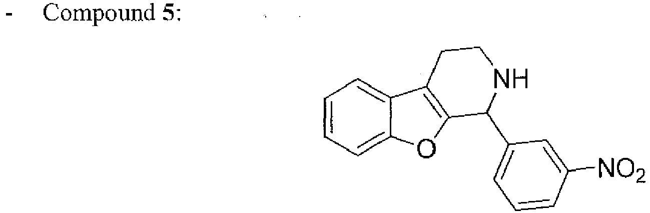

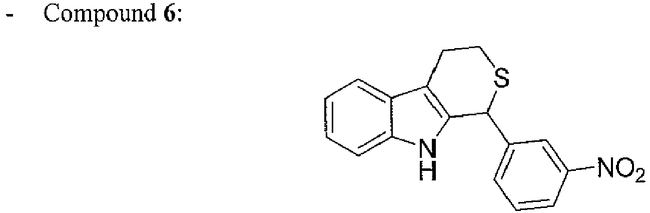

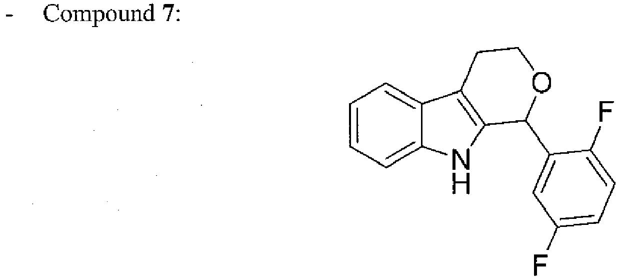

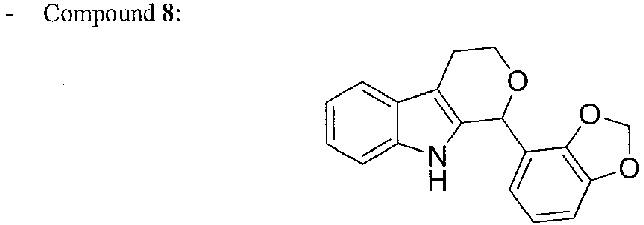

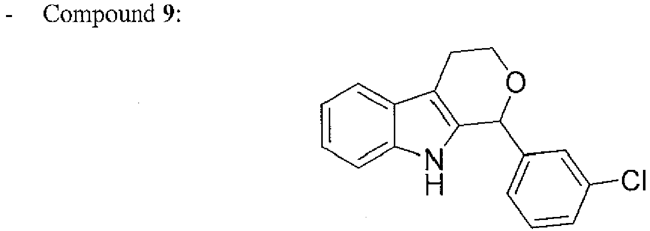

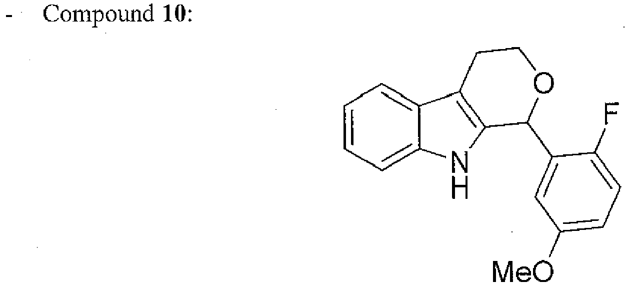

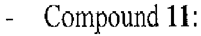

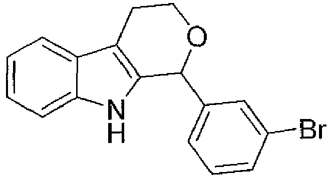

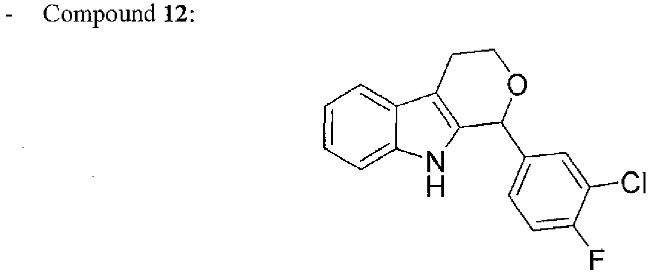

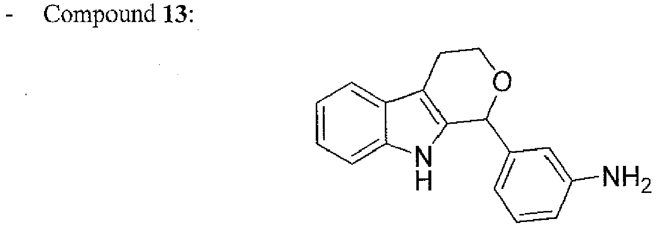

These particular compounds may be used as medicaments, and more particularly for the same uses as mentioned above:
- for the inhibition of sodium iodide symporter (NIS), and the reduction of iodine transport and/or accumulation into NIS-expressing cells,
- for the *in vivo* diagnosis of NIS pathologies by functional imaging,
- for radioiodide decontamination of humans or animals after exposure to radioactive iodine species,
- for the prevention and/or the treatment of thyroid disorders, and more particularly of hyperthyroidism triggered by iodine overload, thyrotoxicosis, thyroiditis and toxic nodular goiter,
- for the prevention and/or the treatment of cancers, and more particularly of thyroid and breast cancers,
- for the prevention and/or the treatment of autoimmune diseases, and more particularly of Hashimoto and Basedow-Graves' diseases.

In addition to the above provisions, the invention also comprises other provisions which will become clear from the description which follows, which refers to examples evaluating the effects of structural variations of compounds of formula (I) on iodide uptake in FRTL5 cells by measuring the IC₅₀ values of such compounds.

### EXAMPLES:

### I- Synthetic procedure

### I- 1) General methods for chemical syntheses and characterization

Reagents and solvents were from Sigma-Aldrich without further purification. Flash chromatography was performed on a CombiFlash Rf system (Teledyne Isco) using normal phase Redisep (Teledyne Isco) or SNAP (Biotage) cartridges. The HPLC-MS analysis was performed on a system equipped with a binary gradient solvent delivery system (2525, Waters), a photodiode array detector (2996, Waters; 200-400 nm) and an evaporative light-scattering detector (PL-ELS 1000, Polymer Laboratory). This system was coupled to an electrospray ionization Micromass-ZQ spectrometer (Waters) operating in both positive and negative mode. Each compound (7-10 µg) was applied to a 100 x 4.6 mm (3.5 µm) C18-XBridge (Waters) equilibrated with acetonitrile/water/formic acid = 5/95/0.1 (1 mL/min). Samples were eluted by increasing acetonitrile to 100% (8 min), then 100% (13 min). ¹H, ¹³C and ¹⁹F NMR spectra were recorded on a Bruker Avance DPX 400 spectrometer operating at 400 MHz (¹H), 100 MHz (¹³C) and 160 MHz (¹⁹F). The chemical shifts (δ) were expressed in ppm. High-resolution mass spectra (HRMS) were performed on the Imagif platform (CNRS - Gif sur Yvette, France), and recorded on a ESI/TOF LCP premier XE mass spectrometer (Waters) using flow injection analysis mode.

### I- 2) Synthetic preparation and date analysis of Compounds 4-13

The identity of compounds 4-**13** was verified by MS, HRMS, ¹H, ¹³C NMR, and ¹⁹F NMR when appropriate. The purity of all compounds tested was found to exceed 95% using a high-performance liquid chromatography (HPLC) system.

*Preparation of 1-(3-nitrophenyl)-1,3,4,9-tetrahydropyrono[3,4-b]indole (4).*

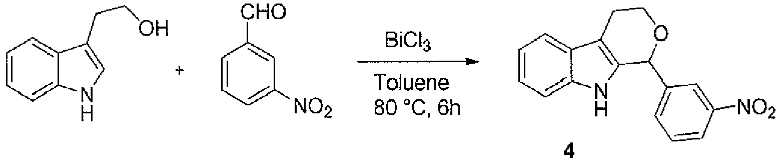

3-(2-Hydroxyethyl)indole (3.22 g, 20 mmol) and 3-nitrobenzaldehyde (3.0 g, 20 mmol) were dissolved in anhydrous toluene (100 mL) under argon atmosphere. Bismuth trichloride (6.30 g, 20 mmol) was added and the mixture was stirred at 80°C for 6 h. Solvent evaporation under reduced pressure followed by chromatography on silica gel (cHex/AcOEt 94/6) afforded 4 as a yellow solid (1.18 g, 20%).

¹H RMN (400 MHz, CDCl₃) *δ* 2.84-2.88 (m, 1H), 3.08-3.14 (m, 1H), 3.97-4.01 (m, 1H), 4.29-4.34 (m, 1H), 5.91 (s, 1H), 7.13-7.20 (m, 2H), 7.26 (d, *J* = 7.2 Hz, 1H), 7.49 (bs, 1H), 7.53-7.58 (m, 2H), 7.72 (d, *J*= 8.0 Hz, 1H), 8.21 (d, *J* = 8.0 Hz, 1H), 8.27 (s, 1H).

¹³C RMN (100 MHz, CDCl₃) *δ* 22.4, 65.1, 75.3, 109.6, 111.3, 118.7, 120.2, 122.7, 123.4, 124.7, 127.1, 130.1, 132.2, 134.7, 136.5, 142.1, 148.7.

HPLC: *t*_{R} = 8.78 min.

MS: *m*/*z* 293 ([M-H]⁻).

HRMS-ESI-TOF : *m*/*z* calculated 293.0926, found 293.0925 ([M-H]⁻).

### Preparation of 1-(3-nitrophenyl)-1,2,3,4-tetrahydro[1]benzofuro[2,3-c]pyridine (5).

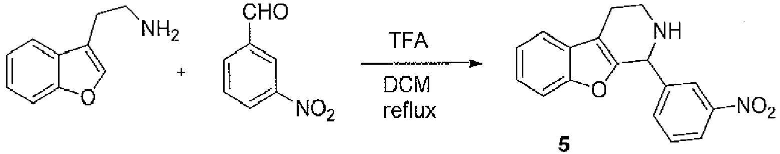

3-Nitrobenzaldehyde (111.8 mg, 0.74 mmol) and 2-(benzofuran-3-yl)ethanamine (119.3 mg, 0.74 mmol) were dissolved in anhydrous dichloromethane (5 mL). The mixture was cooled to 0°C before trifluoroacetic acid (73.4 µL, 0.96 mmol) was slowly added via syringe. The mixture was stirred for 10 min at 0°C, and then refluxed for 14 h. For completion of the reaction, extra trifluoroacetic acid (76.5 µL, 1.0 mmol) was added and the mixture was further refluxed for 24 h. Solvent was removed under reduced pressure. The resulting residue was triturated in diethyl ether (15 mL), filtered and dried to yield the TFA salt of 5 as a brown solid (105 mg, 48%).

¹H RMN (400 MHz, CDCl₃) *δ* 3.04-3.17 (m, 2H), 3.48-3.60 (m, 2H), 6.28 (s, 1H), 7.36-7.38 (m, 2H), 7.56-7.58 (m, 1H), 7.73-7.75 (m, 1H), 7.79-7.86 (m, 2H), 8.38-8.40 (m, 2H), 9.88 (bs, 1H), 10.15 (bs, 1H).

¹³C RMN (100 MHz, CDCl₃) *δ* 7.9, 54.4, 111.6, 113.9, 120.0, 123.5, 124.8, 125.1, 125.4, 126.3, 130.7, 134.7, 136.4, 145.5, 147.9, 154.4.

HPLC: *t*_{R} = 5.75 min.

MS: *m*/*z* 295 ([M+H]⁺).

*Preparation of 1-(3-nitrophenyl)-1,3,4,9-tetrahydrothiopyrano[3,4-b]indole (6).*

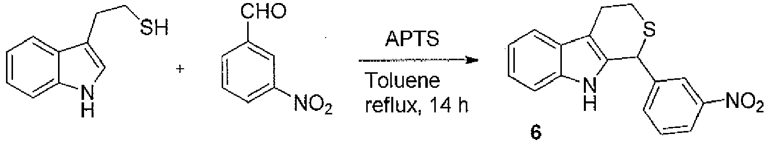

Thiotryptophol was synthesized as described in the publication I. Jirkovsky et al., J. Het. Chem., 1975, 5, 937-940.

Thiotryptophol (200.3 mg, 1.13 mmol) and 3-nitrobenzaldehyde (204 mg, 1.35 mmol) were dissolved in anhydrous toluene under argon atmosphere and stirred at 70°C for 30 min. *Para*-toluenesulfonic acid monohydrate (106.5 mg, 0.56 mmol) was then added and the mixture was further refluxed for 14 h. Solvent evaporation under reduced pressure followed by chromatography on silica gel (cHex/AcOEt 10/0 to 7/3) afforded 6 as a white solid (109 mg, 31%).

¹H RMN (400 MHz, CDCl₃) *δ* 2.95-3.08 (m, 2H), 3.11-3.26 (m, 2H), 5.30 (s, 1H), 7.14-7.26 (m, 3H), 7.48 (t, *J*= 8.2 Hz, 1H), 7.55-7.59 (m, 3H), 8.13-8.15 (m, 2H).

¹³C RMN (100 MHz, CDCl₃) *δ* 23.5, 25.6, 41.0, 111.0, 112.9, 118.7, 120.1, 123.0, 123.1, 123.3, 127.6, 129.4, 129.9, 134.4, 135.4, 144.1, 148.7.

HPLC: *t*_{R} = 9.63 min.

MS: *m*/*z* 311 ([M+H]⁺).

HRMS-ESI-TOF: *m*/*z* calculated 309.0698, found 309.0697 ([M-H]⁻).

### Preparation of 1-(2,5-difluorophenyl)-1,3,4,9-tetrahydropyrano[3,4-b]indole (7).

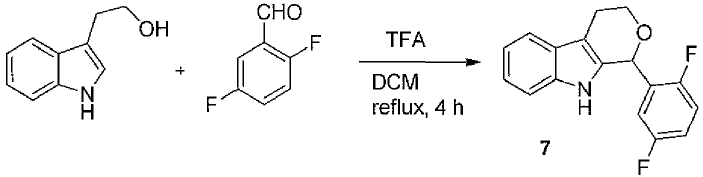

To a solution of 2,5-difluorobenzaldehyde (264 mg, 1.86 mmol) in dry dichloromethane (10 mL) was added a solution of 3-(2-hydroxyethyl)indole (200 mg, 1.24 mmol) in dry dichloromethane (4 mL). This solution was placed under argon. Then a solution of trifluoroacetic acid (28.5 µL, 0.37 mmol) in dry dichloromethane (1 mL) was slowly added. The resulting mixture was stirred for 20 min at room temperature and further refluxed for 4 h. Solvent evaporation under reduced pressure followed by chromatography on silica gel (cHex/AcOEt 94/6) afforded 7 as a white solid (67 mg, 19%).

¹H RMN (400 MHz, CDCl₃) *δ* 2.80-2.86 (m, 1H), 3.05-3.13 (m, 1H), 3.98-4.05 (m, 1H), 4.34-4.38 (m, 1H), 6.20 (s, 1H), 6.98-7.03 (m, 1H), 7.07-7.13 (m, 4H), 7.29 (d, *J* = 7.6 Hz, 1H), 7.55 (d, *J*= 7.6 Hz, 1H), 7.67 (bs, 1H).

¹³C RMN (100 MHz, CDCl₃) *δ* 22.4, 65.2, 70.1 (d, *J* = 3.1 Hz), 109.3, 111.3, 116.0 (dd, *J* = 25.1, 4.6 Hz), 116.5-117.0 (m), 118.6, 120.0, 122.5, 127.0, 129.2 (dd, *J* = 16.0, 6.9 Hz), 132.1, 136.3, 156.5 (dd, *J*= 240.0, 2.3 Hz), 159.2 (dd, *J*= 242.4, 2.3 Hz).

¹⁹F RMN (160 MHz, CDCl₃) *δ* -125.36 (d, *J* = 7.5 Hz), -117.44 (d, *J* = 7.5 Hz).

HPLC: *t*_{R} = 9.35 min.

MS: *m*/*z* 286 ([M+H]⁺).

HRMS-ESI-TOF: *m*/*z* calculated 284.0887, found 284.0885 ([M-H]⁻).

### Preparation of 1-(benzo[d][1,3]dioxol-4-yl)-1,3,4,9-tetrahydropyrano[3,4-b]indole (8).

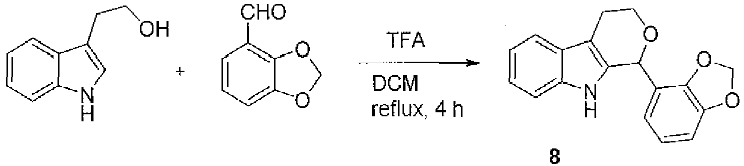

To a solution of 2,3-(methylenedioxy)benzaldehyde (279 mg, 1.86 mmol) in dry dichloromethane (10 mL) was added a solution of 3-(2-hydroxyethyl)indole (200 mg, 1.24 mmol) in dry dichloromethane (4 mL). This solution was placed under argon. Then a solution of trifluoroacetic acid (28.6 µL, 0.37 mmol) in dry dichloromethane (1 mL) was slowly added. The resulting mixture was stirred for 20 min at room temperature and further refluxed for 4 h. Solvent evaporation under reduced pressure followed by chromatography on silica gel (cHex/AcOEt 94/6) afforded **8** as a white solid (73 mg, 20%).

¹H RMN (400 MHz, CDCl₃) *δ* 2.80-2.87 (m, 1H), 3.03-3.07 (m, 1H), 3.99-4.05 (m, 1H), 4.34-4.37 (m, 1H), 5.99 (d, *J* = 1.2 Hz, 1H), 6.08 (s, 1H), 6.09 (d, *J* = 1.2 Hz, 1H), 6.79-6.83 (m, 3H), 7.11-7.17 (m, 2H), 7.28 (d, *J* = 7.6 Hz, 1H), 7.55 (d, *J* = 7.6 Hz, 1H), 7.77 (bs, 1H).

¹³C RMN (100 MHz, CDCl₃) *δ* 22.5, 65.1, 70.1, 101.4, 109.0, 109.1, 111.2, 118.5, 119.8, 121.2, 121.6, 122.2, 122.2, 127.1, 132.9, 136.2, 145.6, 147.8.

HPLC: *t*_{R} = 8.97 min.

MS : *m*/*z* 294 ([M+H]⁺).

HRMS-ESI-TOF: *m*/*z* calculated 292.0974, found 292.0981 ([M-H]⁻).

### Preparation of 1-(3-chlorophenyl]-1,3,4,9-tetrahydropyrano[3,4-b]indole (9).

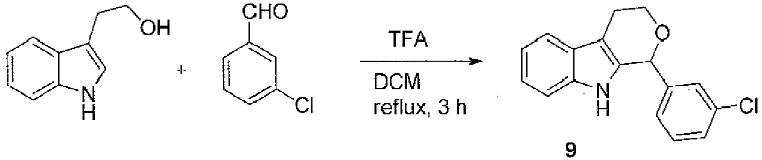

To a solution of 3-chlorobenzaldehyde (262 mg, 1.86 mmol) in dry dichloromethane (10 mL) was added a solution of 3-(2-hydroxyethyl)indole (300 mg, 1.86 mmol) in dry dichloromethane (4 mL). This solution was placed under argon. Then a solution of trifluoroacetic acid (42.7 µL, 0.56 mmol) in dry dichloromethane (1 mL) was slowly added. The resulting mixture was stirred for 20 min at room temperature and further refluxed for 3 h. Solvent evaporation under reduced pressure followed by chromatography on silica gel (cHex/AcOEt 94/6) afforded **9** as a white solid (121 mg, 23%).

¹H RMN (400 MHz, CDCl₃) *δ* 2.82-2.88 (m, 1H), 3.07-3.15 (m, 1H), 3.96-4.02 (m, 1H), 4.29-4.34 (m, 1H), 5.76 (s, 1H), 7.14-7.21 (m, 2H), 7.23-7.31 (m, 2H), 7.32-7.39 (m, 3H), 7.52 (bs, 1H), 7.58 (d, *J*= 7.2 Hz).

¹³C RMN (100 MHz, CDCl₃) *δ* 22.4, 65.1, 75.7, 109.2, 111.3, 118.6, 120.0, 122.4, 125.7, 127.1, 128.7, 129.3, 130.3, 133.0, 135.0, 136.3, 141.8.

HPLC: *t*_{R} = 9.65 min.

MS : *m*/*z* 284 ([M+H]⁺).

HRMS-ESI-TOF: *m*/*z* calculated 282.0686, found 282.0693 ([M-H]⁻).

### Preparation of 1-(2-fluoro-5-methoxyphenyl)-1,3,4,9-tetrahydropyrano[3,4-b]indole (10).

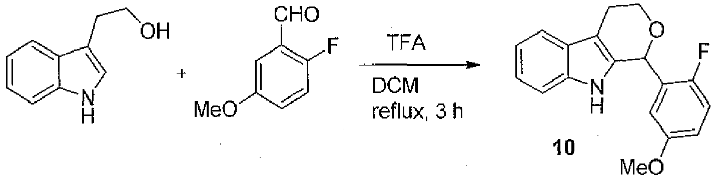

To a solution of 2-fluoro-5-methoxybenzaldehyde (287 mg, 1.86 mmol) in dry dichloromethane (10 mL) was added a solution of 3-(2-hydroxyethyl)indole (300 mg, 1.86 mmol) in dry dichloromethane (4 mL). This solution was placed under argon. Then a solution of trifluoroacetic acid (42.7 µL, 0.56 mmol) in dry dichloromethane (1 mL) was slowly added. The resulting mixture was stirred for 20 min at room temperature and further refluxed for 3 h. Solvent evaporation under reduced pressure followed by chromatography on silica gel (cHex/AcOEt 94/6) afforded **10** as a yellow solid (105 mg, 19%).

¹H RMN (400 MHz, CDCl₃) *δ* 2.83-2.87 (m, 1H), 3.10-3.18 (m, 1H), 3.71 (s, 3H), 4.01-4.07 (m, 1H), 4.39-4.43 (m, 1H), 6.22 (s, 1H), 6.83-6.87 (m, 1H), 6.91-6.93 (m, 1H), 7.07-7.12 (m, 1H), 7.14-7.21 (m, 2H), 7.25.7.28 (m, 1H), 7.57-7.59 (m, 1H), 7.81 (bs, 1H).

¹³C RMN (100 MHz, CDCl₃) *δ* 22.4, 56.0, 65.4, 69.2 (d, *J* = 3.2 Hz), 109.0, 111.3, 113.6 (d, *J* = 3.7 Hz), 115.6 (d, *J*= 8.0 Hz), 116.3 (d, *J* = 23.7 Hz), 118.5, 119.9, 122.2, 127.1, 127.8 (d, *J* = 15.0 Hz), 132.9, 136.2, 155.4 (d, *J*= 236.7 Hz), 156.3 (d, *J* = 1.9 Hz).

¹⁹F RMN (160 MHz, CDCl₃) *δ* -130.22.

HPLC: *t*_{R} = 9.08 min.

MS : *m*/*z* 298 ([M+H]⁺).

HRMS-ESI-TOF: *m*/*z* calculated 296.1087, found 296.1082 ([M-H]⁻).

### Preparation of 1-(3-bromophenyl)-1,3,4,9-tetrahydropyrano[3,4-b]indole (11).

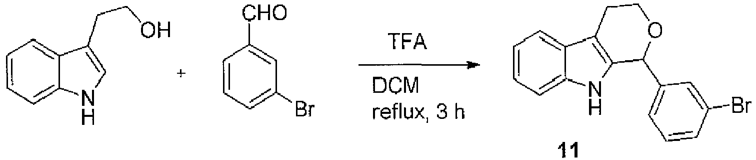

To a solution of 3-bromobenzaldehyde (344 mg, 1.86 mmol) in dry dichloromethane (10 mL) was added a solution of 3-(2-hydroxyethyl)indole (300 mg, 1.86 mmol) in dry dichloromethane (4 mL). This solution was placed under argon. Then a solution of trifluoroacetic acid (42.7 µL, 0.56 mmol) in dry dichloromethane (1 mL) was slowly added. The resulting mixture was stirred for 20 min at room temperature and further refluxed for 3 h. Solvent evaporation under reduced pressure followed by chromatography on silica gel (cHex/AcOEt 94/6) afforded **11** as a white solid (116 mg, 19%).

¹H RMN (400 MHz, CDCl₃) *δ* 2.83-2.88 (m, 1H), 3.07-3.15 (m, 1H), 3.95-4.02 (m, 1H), 4.29-4.34 (m, 1H), 5.75 (s, 1H), 7.15-7.22 (m, 2H), 7.24-7.28 (m, 2H), 7.32 (d, *J*= 7.6 Hz, 1H), 7.50-7.55 (m, 3H), 7.59 (d, *J*= 7.2 Hz, 1H).

¹³C RMN (100 MHz, CDCl₃) *δ* 22.4, 65.0, 75.6, 109.2, 111.3, 118.6, 120.0, 122.4, 123.1, 127.1, 127.2, 130.6, 131.5, 132.2, 132.9, 136.3, 142.0.

HPLC: *t*_{R} = 9.82 min.

MS : *m*/*z* 328(50) and 330(50) ([M+H]⁺).

HRMS-ESI-TOF: *m*/*z* calculated 326.0181, found 326.0192 ([M-H]⁻).

### Preparation of 1-(3-chloro-4-fluorophenyl)-1,3,4,9-tetrahydropyrano[3,4-b]indole (12).

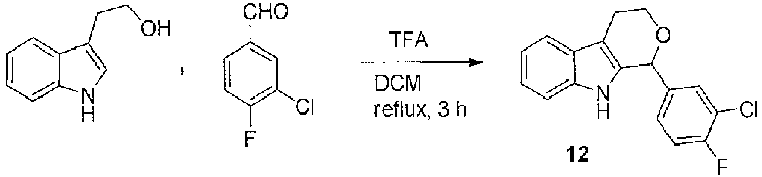

To a solution of 3-chloro-4-fluorobenzaldehyde (295 mg, 1.86 mmol) in dry dichloromethane (10 mL) was added a solution of 3-(2-hydroxyethyl)indole (300 mg, 1.86 mmol) in dry dichloromethane (4 mL). This solution was placed under argon. Then a solution of trifluoroacetic acid (42.7 µL, 0.56 mmol) in dry dichloromethane (1 mL) was slowly added. The resulting mixture was stirred for 20 min at room temperature and further refluxed for 3 h. Solvent evaporation under reduced pressure followed by chromatography on silica gel (cHex/AcOEt 94/6) afforded **12** as a yellow solid (90 mg, 16%).

¹H RMN (400 MHz, CDCl₃) *δ* 2.84-2.89 (m, 1H), 3.07-3.15 (m, 1H), 3.96-4.02 (m, 1H), 4.27-4.32 (m, 1H), 5.75 (s, 1H), 7.13-7.23 (m, 3H), 7.24-7.28 (m, 2H), 7.44 (dd, *J*= 7.2, 2.0 Hz, 1H), 7.57 (bs, 1H), 7.60 (d, *J*= 7.6 Hz).

¹³C RMN (100 MHz, CDCl₃) *δ* 22.4, 65.0, 75.1, 109.5, 111.3, 117.1 (d, *J*= 21.1 Hz), 118.7, 120.1, 121.7 (d, *J =* 17.8 Hz), 122.5, 127.1, 128.4 (d, *J* = 7.6 Hz, 130.9, 132.8, 136.4, 137.0 (d, *J*= 3.6 Hz), 158.5 (d, *J*= 249.1 Hz).

¹⁹F RMN (160 MHz, CDCl₃) *δ* -114.80.

HPLC: *t*_{R} = 9.80 min.

MS : *m*/*z* 302 ([M+H]⁺).

HRMS-ESI-TOF: *m*/*z* calculated 300.0591, found 300.0597 ([M-H]⁻).

### Preparation of 1-(3-aminophenyl)-1,3,4,9-tetrahydropyrano[3,4-b]indole (13).

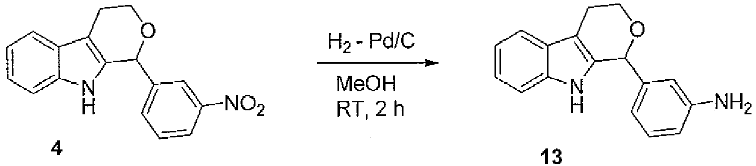

To a solution of 1-(3-nitrophenyl)-1,3,4,9-tetrahydropyrano[3,4-b]indole 4 (500 mg, 1.70 mmol) in methanol (80 mL) was added Pd/C 10% w/w (181 mg, 0.1 eq). This solution was placed under H₂ atmosphere (1.1 bar) and stirred for 2 h at room temperature. The suspension was filtered on a Celite pad. The filtrate was evaporated and chromatographied on silica gel (cHex/AcOEt 94/6) to afford 13 as an orange solid (391 mg, 87%).

¹H RMN (400 MHz, CDCl₃) *δ* 2.78-2.82 (m, 1H), 3.02-3.06 (m, 1H), 3.89-393 (m, 1H), 3.95 (bs, 2H), 4.26-4.28 (m, 1H), 5.60 (s, 1H), 6.61 (s, 1H), 6.70 (d, *J* = 7.2 Hz, 1H), 6.82 (d, *J*= 7.6 Hz, 1H), 7.02-7.10 (m, 4H), 7.55-7.57 (m, 1H), 7.93 (bs, 1H).

¹³C RMN (100 MHz, CDCl₃) *δ* 22.5, 65.2, 76.4, 108.7, 111.2, 115.1, 116.1, 118.5, 119.1, 119.8, 122.1, 127.2, 130.0, 134.0, 136.2, 140.9, 146.6.

HPLC: *t*_{R} = 6.58 min.

MS : *m*/*z* 265 ([M+H]⁺).

HRMS-ESI-TOF: *m*/*z* calculated 263.1184, found 263.1188 ([M-H]⁻).

### II- Biological results

The synthesized compounds were evaluated for their ability to inhibit iodide entrapment in FRTL5 cells using a rodiodide uptake assay. The IC₅₀ values were measured in at least two independent experiments. The Compound **2 (ITB-2)** was used as the reference compound (IC₅₀ = 0.4 µM), and sodium perchlorate as an assay control (IC₅₀ = 0.1 µM).

### II- 1) Protocols for biological evaluation

### Biological evaluation of the synthesized compounds:

The biological activity of each compound was determined in FRTL5 cells, using a 24-well plate format radioiodide uptake assay. Each experiment was run in quadruplicate and at least twice independently. Compound potency was expressed as IC₅₀, the concentration of compound necessary to achieve 50% inhibition of iodide uptake. NaClO₄ and the Compound 2 were tested as assay controls.

### Cell culture and cell seeding in 24-well plates:

FRTL5 cells were cultured as described in the publication of T. Mosmann, J. Immunol. Methods, 1983, 65, 55-63.

FRTL5 cells were cultured in Coon's modified F12 medium supplemented with 5% heat-inactivated fetal bovine serum (Invitrogen), 2 mM L-glutamine, 100 U/mL penicillin, 0.1 mg/mL streptomycin, 10 µg/mL insulin, 10 nM hydrocortisone, 10 ng/mL Gly-His-Lys acetate, 1 mU/mL TSH, 5 µg/mL transferrin at 37°C and 5% CO₂. For iodide uptake assays, 1.25 mL of FRTL5 cells with density of 250,000 cells/mL were dispensed in each well of clear flat-bottomed 24-well polystyrene microplates (BD Falcon 3047), and further cultured until confluence reached 70-90% (3-4 days).

### Chemicals and solutions:

All chemicals were from Sigma-Aldrich unless otherwise stated.

Uptake buffer: Hank's balanced salt solution (HBSS) supplemented with Hepes (10 mM final).

[125-I]-NaI (Perkin Elmer) adjusted to 1 mCi/mL with H₂O.

NaI at 2 mM in H₂O.

Compounds 2, 4-13 at 20 mM in DMSO and NaClO₄ at 20 mM in H₂O.

Lysis buffer: SDS 0.1% and Triton 0.5% in H₂O.

### Working solutions for one 24-well plate:

Prepare ∼1.5 mL/plate of an intermediate "10-fold" radioiodide uptake buffer solution: NaI (100 µM) and [125-I]-NaI (20 µCi/mL) in uptake buffer (HBSS/Hepes).

For each concentration of compound tested, prepare ∼2.5 mL of compound solutions in uptake buffer (HBSS/Hepes 10 mM) at 1.12 fold the final concentration, so that 450 µL of this solution plus 50 µL of radioiodide uptake buffer makes the desired final concentration (NaI 10µM final, 1 µCi/well).

### Radioiodide uptake assay for one 24-well plate:

The procedure is performed at 20 ± 2 °C in a well ventilated hood. Culture medium of FRTL5 cells is removed by aspiration. Compound solutions are added to the 24-well plate in quadruplicate (450 µL/well) immediately followed by [125I]-NaI radioiodide uptake buffer (50 µL/well, 10 µM final, 1 µCi/well). The 24-well plate is left to stand for 60 min. Radioactive supernatants are removed by aspiration and the cell are washed twice with 500 µL of cold uptake buffer (HBSS/Hepes at 4°C). Finally, the residual supernatant is removed by aspiration and 500 µL of lysis buffer (SDS 0.1%, triton 0.5%) is added to each well. The 24-well plate is gently agitated for 60-120 min. The radioactive cell lysates are collected and placed into scintillation vials. Scintillation cocktail (Ultima gold LLT, Perkin Elmer) is added to each vial (4 mL) and the radioactivity is measured (Wallac 1409). For IC₅₀ determination, mean DPM values were fitted by non-linear regression (least square) to the four-parameter sigmoidal Hill equation.

### II- 2) Results

**Table 1: Inhibitory activity (IC₅₀) of Compounds 4-13 against iodide uptake in FRTL5 cells**

| | Compounds | IC₅₀ (nM) |
|---|---|---|
| 4 | 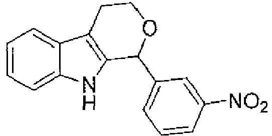 | 5.3 |
| 5 | 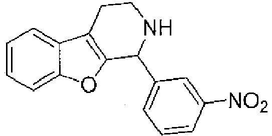 | 200 |
| 6 | 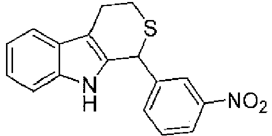 | 140 |
| 7 | 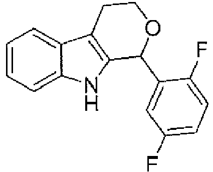 | 170 |
| 8 | 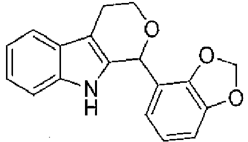 | 0.41 |
| 9 | 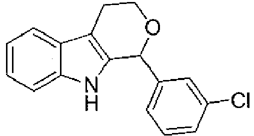 | 140 |
| 10 | 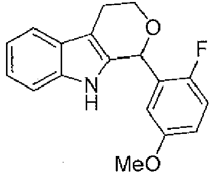 | 57 |
| 11 | 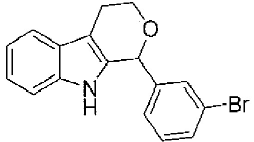 | 170 |
| 12 | 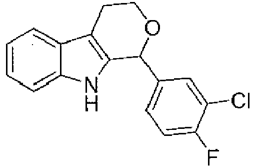 | 200 |
| 13 | 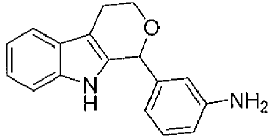 | 1.1 |

IC₅₀ values are averaged from two to four independent experiments. A standard deviation of 2-fold was judged acceptable. NaClO4 and Compound **2** were tested as assay controls.

## Claims

1. A compound of formula (I) below: 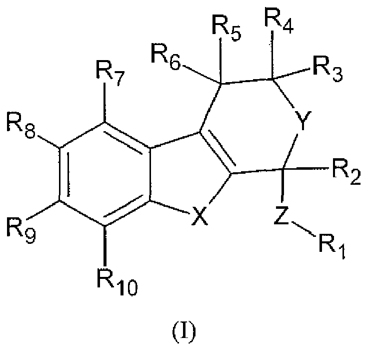 or a pharmaceutically acceptable salt thereof, wherein:
● X = O, S or NR, wherein R is a hydrogen atom or an optionally substituted C₁-C₂₀ linear or branched alkyl chain or an optionally substituted C₃-C₇ cycloalkyl chain, said alkyl or cycloalkyl chain possibly including one or more heteroatoms or carbonyl functions,
● Y = O, S or NH,
● Z is a simple bond or an optionally substituted C₂-C₆ linear or branched alkyl or alkenyl chain or an optionally substituted C₃-C₆ cycloalkyl chain,
● R₁ is an optionally substituted aromatic ring,
● R₂, R₃, R₄, R₅, R₆, R₇, R₈, R₉ and R₁₀, identical or different, are selected from hydrogen atom, optionally substituted C₁-C₂₀ linear or branched alkyl, alkoxy, alkenyl, alkynyl, cycloalkyl, aryl, arylalkyl, heteroalkyl, heteroaryl and heteroarylalkyl groups, and wherein at least one of X or Y is O or S,
for its use as a medicament.

2. The compound of formula (I) for its use according to Claim 1, wherein X = NH.

3. The compound of formula (I) for its use according to Claim 1 or Claim 2, wherein Y=O.

4. The compound of formula (I) for its use according to Claims 1 to 3, wherein Z is a simple bond or a C₂ double bond, and preferably Z is a simple bond.

5. The compound of formula (I) for its use according to Claims 1 to 4, wherein R₁ is an optionally substituted aryl or heteroaryl ring.

6. The compound of formula (I) for its use according to Claim 5, wherein R₁ is a 2,3-(methylenedioxy)-phenyl group or a phenyl ring substituted with at least one halogen atom, nitro (NO₂), amino (NH₂) or C₁-C₆ alkoxy group.

7. The compound of formula (I) for its use according to Claims 1 to 6, wherein R₂, R₃, R₄, R₅, R₆, R₇, R₈, R₉ and R₁₀ are hydrogen atoms.

8. The compound of formula (I) for its use according to Claims 1 to 7, for the inhibition of sodium iodide symporter (NIS).

9. The compound of formula (I) for its use according to Claims 1 to 7, for the reduction of iodine transport and/or accumulation into NIS-expressing cells.

10. The compound of formula (I) for its use according to Claims 1 to 7, for the *in vivo* diagnosis of NIS pathologies by functional imaging.

11. The compound of formula (I) for its use according to Claims 1 to 7, for radioiodide decontamination after exposure to radioactive iodine species.

12. The compound of formula (I) for its use according to Claims 1 to 7, for the prevention and/or the treatment of thyroid disorders, and more particularly of hyperthyroidism triggered by iodine overload, thyrotoxicosis, thyroiditis and toxic nodular goiter.

13. The compound of formula (I) for its use according to Claims 1 to 7, for the prevention and/or the treatment of cancers, and more particularly of thyroid and breast cancers.

14. The compound of formula (I) for its use according to Claims 1 to 7, for the prevention and/or the treatment of autoimmune diseases, and more particularly of Hashimoto and Basedow-Graves' diseases.

15. A pharmaceutical composition comprising at least one compound of formula (I) as defined according to Claims 1 to 7 as an active principle, and at least one pharmaceutically acceptable excipient.

16. A compound of formula (I) below:
